# EUROPEAN PATENT SPECIFICATION

(11) **EP 1 646 345 B1**
(45) Date of publication and mention of the grant of the patent: **19.03.2014**
(21) Application number: 04749665.8
(22) Date of filing: 31.03.2004
(51) Int. Cl.: A61F 13/15, A41B 9/00

(54) **DISPOSABLE UNDERGARMENT HAVING A DISCONTINUITY AND METHODS FOR THE MANUFACTURE THEREOF**
EINWEG-UNTERBEKLEIDUNG MIT EINER DISKONTINUITÄT UND VERFAHREN ZUR HERSTELLUNG DAFÜR
SOUS-VETEMENT JETABLE PRESENTANT UNE DISCONTINUITE ET PROCEDES DE PRODUCTION ASSOCIES

(30) Priority: 22.07.2003 US 624333; 22.07.2003 US 624660
(43) Date of publication of application: 19.04.2006
(73) Proprietor: KIMBERLY-CLARK WORLDWIDE, INC., Neenah, WI 54956 (US)
(72) Inventor: VAN GOMPEL, Paul, T., Hortonville, WI 54944 (US); THORSON, Russell, E., Appleton, WI 54914 (US); HUANG, Yung, H., Appleton, WI 54914 (US); WAGNER, Kenneth, J., Greenville, WI 54942 (US); NIEMEYER, Jean, F., Appleton, WI 54913 (US); MUSIL, Judith, A., Appleton, WI 54915 (US); WUDI, Heidi, K., Appleton, WI 54914 (US); BALOGH, Bridget, A., Menasha, WI 54952 (US); MAAS, Cindi, Appleton, WI 54915 (US); VEITH, Jerome, Steve, Menasha, WI 54952 (US)
(74) Representative: Davies, Christopher Robert
(86) International application number: PCT/US2004/010159
(87) International publication number: WO 2005/016200

(56) References cited:
- EP-A- 0 648 482
- EP-A1- 1 350 498
- FR-A- 1 177 805
- US-A- 3 895 629
- US-A- 4 834 737
- US-A- 5 062 840
- US-A- 5 624 422

## Description

### BACKGROUND

The present invention relates generally to disposable undergarments, and in particular, to an undergarment including one or more components with a discontinuity, such as a slit or cutout, and to the methods for the manufacture thereof.

Disposable undergarments can be configured in many different forms. For example, disposable absorbent garments can be configured as a pant-type, pull-on garment, or as a diaper-type product that is drawn up between the legs and fastened about the waist with various fastening systems. Typically, the undergarment includes a body chassis, which is secured to the body of the user. In some embodiments, the disposable garment includes a front and rear body panel connected to an absorbent insert. Often, the absorbent insert is secured to a bodyside surface of the body panels, or is sandwiched between a body panel and an outer cover, which body panels and/or outer cover restrict the ability of the absorbent insert to expand away from the body of the user.

Often, one or more components of the body chassis, such as the outer cover, are made from a single piece of material, with leg openings cut therein, for example by die cutting. The material from the leg cut out, which can account for as much as 20-30% of the total area of the outer cover, typically is waste material, which must be disposed of or recycled. In addition, the size of the leg openings formed by a die cutter in a conventional one-piece outer cover is typically fixed. As such, it can be expensive and time consuming, and reduces the overall flexibility of the manufacturing line, to switch dies and alter the process to manufacture different size garments.

In response to this problem of waste, some garments are configured with front and rear sections formed from a single web that is divided into two nested halves, as disclosed for example in U.S. Patent No. 5,858,151 and Japanese Patent Application 03-176053 A. However; the webs of the U.S. Patent No. 5,858,151 have overlapping crotch portions that are directly secured one to the other. Accordingly, the overall rise of the garment is not readily varied to accommodate different size users, and the range of sizes is limited by the extent of the overlapping regions..

The diaper body of Japanese Patent Application 03-176053 A requires the two webs to be shifted in the machine direction, with a diaper body then positioned over opposed recesses formed in the front and back waistband. The diaper body, however, is secured to the body side surface of the waistbands. As such, the waistbands can restrict the ability of diaper body to expand away from the body of the user.

In addition, some disposable undergarments are made of one or more elastic materials laminated to a non-elastic material. In some garments, slits are provided in one of the layers, e.g., the non-elastic material, to allow the laminate to expand, but the slit does not provide a discontinuity through the entire laminate or the layer of elastic material. In other undergarments, an elastic material, such as a top sheet that extends the length of the garment, is provided with slits that open to permit the passage of exudates therethrough.

A prior art garment, having the features of the preamble of claims 1 and 10, is shown in US 6 264 641 (Van Gompel).

### SUMMARY

There is provided, according to the present invention, a disposable undergarment as claimed in claims 1 and 10 and a method of manufacturing said disposable undergarment as claimed in claims 6 and 12.

The various aspects of the present invention provide significant advantages over other disposable undergarments and methods. For example and without limitation, the cutout formed in the front and rear body panels provides access to the crotch member connected to the garment side of the body panels, e.g., to permit passage of exudates to the crotch member. Conversely, if the crotch member is secured to the body side surface of the body panels, the cutout allows the crotch member to expand away from the body of the user, for example when insulted.

The slit formed in one or both of the front and rear body panels also provides significant advantages. In particular, the slit reduces the force required to elongate the body panels as the garment is applied to the user. In addition, the slit, when opened, provides access to a crotch member secured to the garment side surface of a body panel, or allows the crotch member to expand away from the body of the user if secured to the body side surface of the body panel.

The presently preferred embodiments, together with further advantages, will be best understood by reference to the following detailed description taken in conjunction with the accompanying drawings.

### BRIEF DESCRIPTION OF THE DRAWINGS

FIGURE 1 is a schematic plan view illustration of a method of manufacturing an absorbent garment.
FIGURE 2 is a schematic plan view illustration of an alternative method of manufacturing an absorbent garments.
FIGURE 3 is a schematic plan view illustration of an alternative method of manufacturing an absorbent garment.
FIGURE 4 is a schematic plan view illustration of an alternative method of manufacturing an absorbent garment.
FIGURE 5 is a schematic plan view illustration of an alternative method of manufacturing an absorbent garment, not within the scope of the present invention.
FIGURE 6 is a garment side plan view of a disposable undergarment in an open condition, with a partial cut-away section of a front body panel.
FIGURE 7 is a cross-sectional view of a disposable undergarment taken along line 7-7 of Figure 6.
FIGURE 8 is a body side plan view of an alternative embodiment of a disposable undergarment in an open condition.
FIGURE 9 is a cross-sectional view of a disposable undergarment taken along line 9-9 of Figure 8.
FIGURE 10 is a garment side plan view of an alternative embodiment of a disposable undergarment in an open condition.
FIGURE 11 is a cross-sectional view of a disposable undergarment taken along line 11-I1 of Figure 10.
FIGURE 12 is a plan view of a portion of body panel web in an unstretched condition with a slit formed therein.
FIGURE 13 is a plan view of the portion of body panel web shown in Figure 12 in a stretched condition.
FIGURE 14 is a schematic graphical representation of the force required to elongate a body panel v. the percent elongation thereof for various body panel configuration.
FIGURE 15 is a schematic representation of a test sample and procedure for comparing load v. % strain relationship.
FIGURE 16 is a load v. % strain graph for a first test specimen without a slit.
FIGURE 17 is a load v. % strain graph for a second test specimen without a slit.
FIGURE 18 is a load v. % strain graph for a third test specimen without a slit.
FIGURE 19 is a load v. % strain graph for a fourth test specimen without a slit.
FIGURE 20 is a load v. % strain graph for a fifth test specimen without a slit.
FIGURE 21 is a load v. % strain graph for a first test specimen with a slit.
FIGURE 22 is a load v. % strain graph for a second test specimen with a slit.
FIGURE 23 is a load v. % strain graph for a third test specimen with a slit.
FIGURE 24 is a load v. % strain graph for a fourth test specimen with a slit.
FIGURE 25 is a load v. % strain graph for a fifth test specimen with a slit.
FIGURE 26 is a cross-sectional view of an alternative embodiment of a crotch member secured to a body panel.
FIGURE 27 is a cross-sectional view of an alternative embodiment of a crotch member secured to a body panel.

### DETAILED DESCRIPTION OF THE PRESENTLY PREFERRED EMBODIMENTS

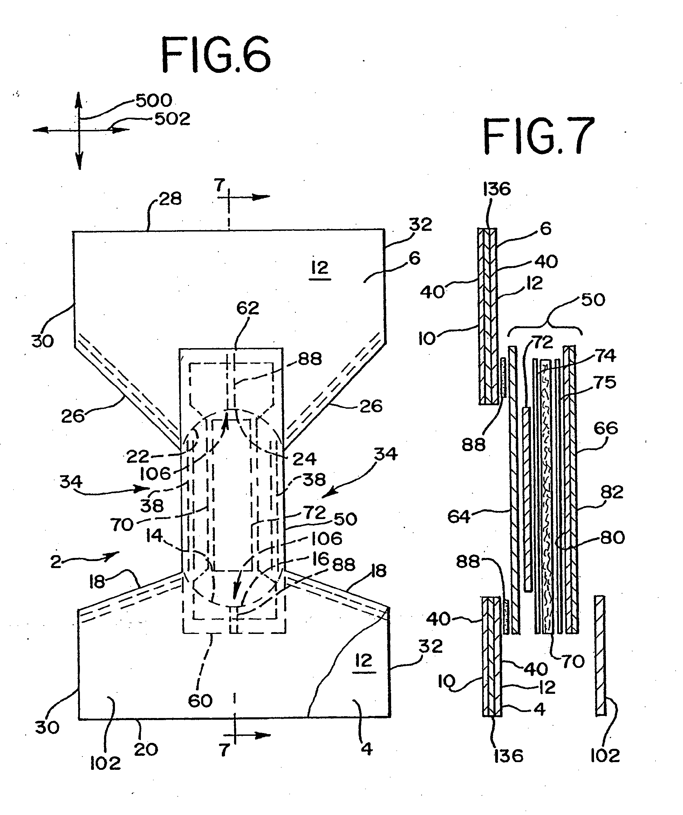

It should be understood that the term "longitudinal," as used herein, means of or relating to length or the lengthwise direction 500. The term "laterally," as used herein, means situated on, directed toward or running from side to side. The term "first direction" generally refers to a path, line or course rather than a vector, and includes and applies equally to opposite orientations along the path, line or course, including for example and without limitation movement along a path, line or course in both directions (as indicated by the bi-directional arrows associated with the longitudinal and lateral directions **500**, **502** in FIG. 6, 8 and 10). Likewise, the term "second direction" generally refers to a path, line or course rather than a vector (not orientation dependent), and includes for example and without limitation movement along a path, line or course in both directions. In one example, the first direction is defined by and refers to one of the longitudinal and lateral directions, while the second direction refers to the other of the longitudinal and lateral directions.

The term "bodyside" should not be interpreted to mean in contact with the body of the user, but rather simply means the side that would face toward the body of the user, regardless of whether an undergarment is actually being worn by the user and regardless of whether there are or may be intervening layers between the component and the body of the user. Likewise, the term "garment side" should not be interpreted to mean in contact with the garments of the user, but rather simply means the side that faces away from the body of the user, and therefore toward any outer garments that may be worn by the user, regardless of whether the undergarment is actually being worn by a user, regardless of whether any such outer garments are actually worn and regardless of whether there may be intervening layers between the component and any outer garment.

The term "machine direction" means the direction of flow as the various members and webs progress along the fabrication line and process. It should be understood that various separate members or webs can each be traveling in a machine direction, but with the various machine directions not necessarily being parallel or oriented in the same direction. For example, a first component such as a web may be traveling a first machine direction, which is substantially perpendicular to the travel of another component, such as an absorbent insert, in a second machine direction.

The term "cross-machine direction" or "cross direction" means the direction substantially perpendicular to the machine direction.

The term "downstream" means that one item is positioned more closely to the output or finished product end of the machine and/or process relative to another item. Conversely, the term "upstream" means that an item is positioned more closely to the input end of the machine or process relative to another item. For example, the output end is downstream of the input end, and vice versa, the input end is upstream of the output end.

The phrases "removeably attached," "removeably attaching," "removeably connected," "removeably engaged," "releasably attached," "releasably connected," or "releasably engaged," and variations thereof, refers to two or more elements being connected or connectable such that the elements tend to remain connected absent a separation force applied to one, both or all of the elements, and where the elements are capable of being separated upon the application of a separation force. The required separation force is typically beyond that encountered while wearing the absorbent garment.

The phrases "fixedly secured," "fixedly engaged," "fixedly attached," "fixedly connected," and variations thereof, refers to two or more elements being connected or connectable such that they are not disconnected or otherwise separated, and are not intended to be separated or disconnected, during the normal operation and use of the absorbent garment.

The term "web" refers to a continuous stream of material, whether made from one or more layers or substrates, and regardless of whether it may have non-continuous, discrete items disposed thereon.

The terms "connecting," "coupled," "attached," and "secured," and variations thereof, broadly covers two or more items being directly connected one to the other, or by way of one or more intervening members or components.

The term "cutout" means that a portion of material is removed. The term "slit" refers to a cut wherein no material is removed.

Referring to FIGS. 6-11, an undergarment 2 includes a first, front body panel 4 and a second, rear body panel 6. The term "body panel" refers to the portion(s) of the undergarment, whether made of one or more layers or substrates or of one or more pieces or components, that is/are fitted circumferentially around at least the waist region of the user, including for example the user's lower back, buttock, hips and abdomen. The first and second body panels each have an inner, bodyside surface **10** and an outer, garment side surface **12**. The first, front body panel **4** has a first edge **14** forming a crotch portion **16** and leg opening portion **18** and a second terminal edge **20** that, in one embodiment, is linear but can assume other shapes. Likewise, the second, rear body panel **6** has a first edge **22** forming a crotch portion **24** and a leg opening portion **26** and a second terminal edge **28**, which is shown linear but can assume other shapes. Each of the first and second body panels has an outboard side edge **30, 32** formed along the outer periphery of the opposite side portions of the first and second body panel. It should be understood that the outboard side edges of the front and rear body panels could have the same or different lengths relative to each other.

One or more, and in one embodiment a plurality, meaning two or more, elastic elements are secured to each of the first and second body panels. In one embodiment, a plurality of elastic elements are spaced across substantially the entire waist portion of the front and rear body panel although they may be spaced across a lesser length. For example, elastic elements can extend along the upper waist portion and along the lower terminal edge defining in part a leg opening **34**. The elastic elements can extend along the entirety of the edges **14, 22**, or along only the leg opening portions **18, 26** thereof.

In one embodiment, the front body panel has a "non-elasticized" area wherein there are no elastic elements, or other elastic or elastomeric backing members, incorporated therein or making up any portion of the thickness or cross-section of the body panel at that area. It should be understood, that in an alternative embodiment, one or more separate waist bands, with or without elastic elements, can be secured to one or both of the rear and front body panels, preferably along the upper terminal edges **20**, **28** thereof. Likewise, one or more separate leg bands can be secured to one or both of the rear and front body panels along the leg open portions **18**, **26** adjacent the leg openings **34**. Alternatively, one or both of the body panels can be formed without any elastic elements.

The various waist and leg elastic elements can be formed from rubber or other elastomeric materials. One suitable material is a LYCRA® elastic material. For example, the various elastic elements can be formed of LYCRA® XA Spandex 540, 740 or 940 decitex T-127 or T-128 elastics available from E.I. duPont De Nemours and Company, having an office in Wilmington, Delaware.

Each body panel **4, 6** is preferably formed as a composite, or laminate material, otherwise referred to as substrates or laminates, with an elastic core **136** sandwiched therebetween. In one embodiment, the elastic core **136** is made of an elastomeric film or nonwoven elastic or stretchable material including for example but not limited to styrenic copolymers of polyisoprene, polybutadiene or polyolefin, copolymers of polyolefins, natural or styrene butadiene rubber, polyurethanes, polyamides, polyesters, and co-extrusions/blends of the aforementioned materials. The elastic core can be formed as a membrane or from a plurality of elastic strands, as described above. In one embodiment, two or more layers **40** are bonded to the elastic core **136**, and/or each other, with various adhesives, such as hot melt, or by other techniques, including for example and without limitation ultrasonic bonding and heat pressure sealing. In one embodiment, the two layers are made of a non-woven material such as a spunbond material, a bonded carded material or other known materials. In this way, the body panels are made of a stretchable/elastic material.

As used herein, the interchangeable terms "stretchable" and "elastic," and variations thereof, refer to a material that can elongate or deform (stretch) in response to the application of a tensile force, and upon removal of the tensile force the material can retract and become shorter. Because of hysteresis, the material may not be able to fully recover or return to its original, pre-stretched length. Thus, a stretchable or elastic material can be stretched and upon relaxing the material, will tend to resume its original shape.

As used herein, the term "extensible" means capable of being extended, and that it provides a selected elongation when subjected to an applied tensile force, while also providing a selected, sustained deformation when subjected to an applied tensile force and then allowed to relax for a selected time period beginning immediately after removal of the tensile force. Preferably the sustained deformation is substantially permanent deformation. The selected elongation and sustained deformation preferably occur at least along the lateral cross-direction of the garment, although it should be understood that it also could occur along the longitudinal direction, or both. In one embodiment, the material is capable of providing an elongation of at least about 1 cm when subjected to a tensile force of 11.8 g/cm, and further provides a substantially permanent deformation of at least about 20% when subjected to a tensile force of 19.70 g/cm and is then allowed to relax under a zero applied stress for a period of 1 minute. Various extensible materials, and other acceptable materials that can be used for the body panels and the absorbent composite, which may include without limitations a retention portion, a topsheet and a backsheet, are described in U.S. Patent No. 6,264,641, entitled Expandable Cover Garment.

A suitable technique for generating a representative tensile-load vs. extension curve, and for determining the amount of elongation and/or retractive force parameters of a selected component or material can employ ASTM Standard Test Method D882 (Tensile Method for Tensile Properties of Thin Plastic Sheeting) dated December 1995, with the following particulars. The "width" of the test sample will be a cross-wise width which can be conveniently obtained from the product being tested, and is desirably about 2 inch (about 5.04 cm). The test sample width is perpendicular to the direction of the tensile force applied during the testing. With regard to the shown configurations, for example, the test sample "width" generally corresponds to the length-wise dimension of the component along the longitudinal direction of the article. The initial separation of the jaws of the tensile tester is 3 inches (7.62 cm), and the moving jaw is moved at a constant rate of 50 mm/min. The moving jaw is stopped at an extension of 50 mm for a period of 10 sec, and then returned back to its initial starting position at a rate of 50 mm/min. The force-extention curve to the complete tension and retraction cycle can be recorded on a conventional computer equipped with commercially available software, such as TestWorks for Windows, version 3.09, which is available from MTS System Corporation, a business having a location at 14000 Technology Drive, Eden Prairie, MN. The obtained data is normalized and reported in appropriate units of force per unit length of sample "width" (e.g. grains-force per inch, or Newtons per inch, or grams-force per centimeter, or Newtons per centimeter).

It should be understood that the body panels can be made of a single layer or substrate of non-woven material, a bi-layer substrate made of non-woven materials without an elastic core, or more than two layers or substrates. Of course, it should be understood that other knitted or woven fabrics, non-woven fabrics, elastomeric materials, polymer films, laminates and the like can be used to form one or more of the body panel layers. The term "non-woven" web or material, as used herein, means a web having a structure of individual fibers or filaments that are interlaid, but not in an identifiable manner and without the aid of textile weaving or knitting, as in a knitted or woven fabric.

In one embodiment, the body panel material can be secured to the elastic core, such as an elastomeric layer or elastic strands or ribbons, which have been elongated and retracted, such that the material is gathered when the elastic element(s) are relaxed. Alternatively, the material can be gathered and laminated to non-elongated elastic elements. In one preferred embodiment, the body panel includes a gathered elastic laminate made from nonwoven base sheets bonded with elongated elastic elements sandwiched therebetween.

In various preferred embodiments, the body panel material may be substantially permeable to air or substantially impermeable to air. The body panel material also may be substantially liquid-permeable or substantially liquid-impermeable. In particular arrangements, the body panel material may be substantially nonelastomeric. In other aspects, the body panels can include an elastomeric material that is elastomerically stretchable at least along one or both of the lateral article width and the longitudinal article length. Examples of such elastomeric composite materials can include a continuous filament stretch bonded laminate (CFSBL), a vertical filament laminate (VFL), neck-bonded-laminate (NBL), a stretch-bonded-laminate (SBL), a necked-stretch bonded laminate (NSBL) or a necked-thermal laminate, or the like, as well as combinations thereof. Exemplary CFSBL, NBL, SBL, and NSBL materials are described in U.S. Patent Nos. 5,226,992, 4,981,747, 4,965,122, 5,336,545, 5,385,775 5,414,470, 4,720,415, 4,789,699, 4,781,966, 4,657,802, 4,652,487, 4,655,760, 5,116,662 and 5,114,781, and 6,323,389. Exemplary VFL materials are described in U.S. Provisional Patent Application Serial Number 60/204,307, filed May 15, 2000 and entitled "Method and Apparatus for Producing Laminated Articles," and PCT application WO 01/88245 A2, both assigned to Kimberly-Clark Worldwide, Inc., the Assignee of the present application. Such laminates can provide an improved combination of cloth-like feel and elastomeric stretchability. The body panels can be composed of materials that are elastic or elastomeric and exhibit biaxial stretch characteristics or lateral/longitudinal stretch characteristics, or which are extensible composites. Additional waist and leg elastic elements can be added to, but are not necessarily required by, the body panels.

In one embodiment, the body panel material is extensible but not elasticized. For example, the body panel can be made of a film or non-woven that is attached, by way of adhesives or thermal bonding, to an extensible non-woven material. Alternatively, the body panel can be made of a low modulus film such as ethylene methyl acrylate (EMA).

As shown in the embodiments of FIGS. 6-11, the entirety of the body panels 4, 6 are elasticized, such that the entirety of each of the body panels can elongate and conform to the body of the user without any substantial spacing between the body panel and the user's body, and without the attendant bulkiness of a non-elasticized material.

In one embodiment, the body panels are breathable, cloth-like, multidirectional nonwoven laminates with stretch or extensible properties. In one embodiment, the non-woven layers are pre-necked, for example between about 10% and about 80%, in the longitudinal direction, which provides extensibility in the longitudinal direction with minimal force.

In one embodiment, the body panel members **4**, **6** are made of non-woven laminates of two layers of longitudinally extensible 0.60 osy (20.35 gm⁻²) polypropylene spunbond material with elongated strands of Lycra® elastic sandwiched between the spunbond layers and thereafter adhesively bonded. In particular, the body panel material is necked in the cross direction. As used herein, the term "necked," and variations thereof, refers to any material that has been constricted in at least one dimension by applying a tensioning force in a direction that is perpendicular to the desired direction of neck-down. Processes that may be used to constrict a material in such a manner include, for example and without limitation, drawing processes. The elastics are then elongated in the machine direction and secured to the body panel material. The elastics are then allowed to retract so as to gather the necked spunbond material in the lateral (machine) direction thereby creating an elastically gathered non-woven body panel with longitudinal extensibility. The term "gather," and variations thereof, as used herein means puckered, or contracted into folds or wrinkles, which should be understood as including micro-pleats.

In this way, the body panel can be elongated in both the longitudinal and lateral direction to conform to the body of the user when the garment is applied thereto. In particular, as the user pulls the garment up over their hips, the non-woven laminate body panels stretch in the lateral direction while the leg regions of the front and rear body panels conform to the crotch and bodylines of the user. At the same time, the body panel material extends in the longitudinal direction to conform to the buttocks and stomach of the user. The extensibility of the body panels follows the natural curvature of user's body to provide conformance thereto. As the body panel extends in the longitudinal direction, the spacing between the laterally extending elastic elements, incorporated in one embodiment, will increase.

The body panel non-woven material is preferably substantially hydrophobic, which may optionally be treated with a surfactant or otherwise processed to impart a desired level of wettability and hydrophilicity. In one particular embodiment of the invention, the body panel is a nonwoven, wire-weave spunbond polypropylene fabric composed of about 1.6 denier (1.6 g per 9,000 m) fibers formed into a web having a basis weight of about 0.6 osy (20.35 gm⁻²). One suitable non-woven material is the Corinth 0.60 osy (20.35 gm⁻²), 1.6 dpf wireweave, nonwettable Metallocene (EXXON ACHIEVE 2854 PP) spunbond material manufactured by Kimberly-Clark Corporation, the assignee of the present application.

The crotch member **50** of the various undergarments connecting the front and rear body panels **4, 6** can be folded such that the side edges **30, 32** of the front and rear body panels **4**, **6** are aligned, whereinafter they can be fixedly secured at a seam to form the leg opening 34. The seam can be formed by bonding, sewing or otherwise attaching the side edges. Alternatively, the product can remain "open," wherein the body panels are releasably secured with one or more fastening members as explained below.

In one embodiment, the garment includes a combination of side edges that are secured to form a seam and fastening members that allow the fit of the undergarment to be adjusted. In other embodiments, the fastening elements are used alone without a seam. For example, in one embodiment, fastening members are preferably attached to the front body panel and extend inboard relative to the outboard side edge of the front body panel from an attachment location, which is preferably spaced inboard from the side edge. A landing member can be formed on or secured to the body panel to receive a refastenable portion of the fastening member. One or more lines of weakness can be provided along the front or rear body panel such that one or both of the body panels are breakable. The lines of weakness can comprise a perforation or other series of cuts, a thinning, breakage or separation of material, or a strip of a different kind of material bridging portions of the body panel that is more easily torn or broken than the other material thereof, which allow a user or the manufacturer to separate portions of the body panel. For example, the undergarment can be broken along the lines of weakness after the garment is applied to a user, or beforehand. In one embodiment, the fastening members are secured to the garment-side surface of the body panel.

It should be understood that, in other embodiments, the fastening members can be secured to the rear body panel and engage the front body panel or, conversely, can be secured to the front body panel and engage the rear body panel, preferably along at least a portion that is not elasticized. In one embodiment, the fastening members are fixedly secured to the outer, garment-side surface of the front and/or rear body panels, and releasably engage the outer, garment-side surface of the front and/or rear body panels, although it should be understood that the fastening members could be fixedly secured to an inner body-side surface of front and/or rear body panels and releasably engage an inner, body-side surface of the front and/or rear body panels.

When incorporated into a disposable absorbent undergarment, the fastening members can include a refastenable portion, such as an array of hook members, adhesives, such as pressure sensitive adhesives, buttons, zippers, snaps and other releasable and reattachable fastening devices. In various embodiments, the fastening member includes one, two or more than two tab members. In one embodiment, the fastening members comprise a carrier member, which is preferably fixedly secured to the side portions of the front body panel with adhesive bonds, sonic bonds, thermal bonds, pinning, stitching or other known types of attachment. In alternative embodiments, the fastening members can be fixedly secured to the rear body panel or to one or both of the front and rear body panels, for example, at the seam, as explained above.

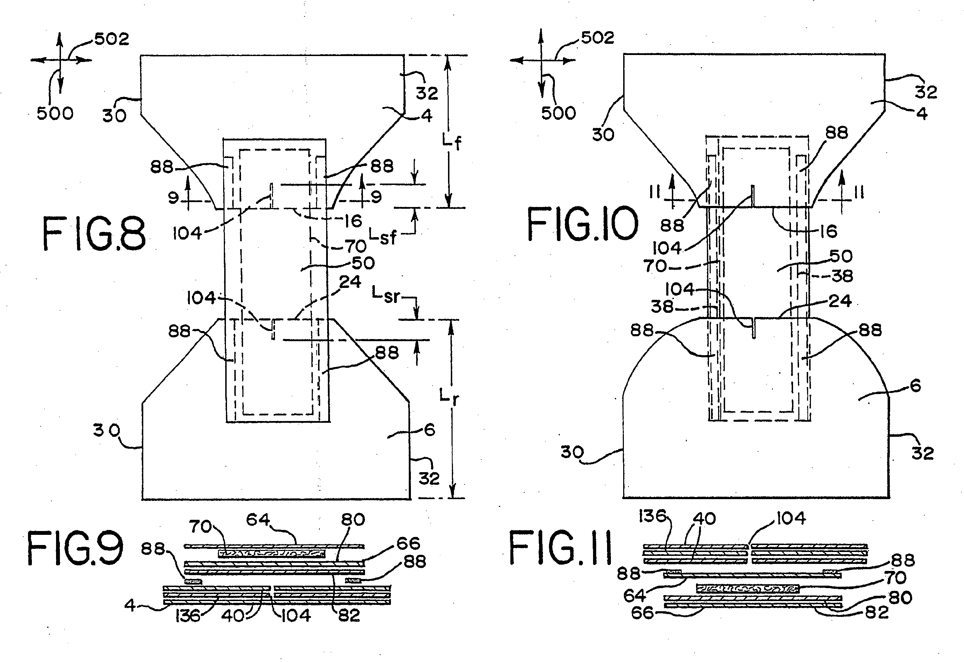

Referring to the embodiments of FIGS. 6-11, the crotch member **50** is formed as a separate subassembly connected to either the bodyside or garment side surface **10, 12** of the body panel members **4, 6**. In either embodiment, the crotch member **50** has first and second opposed terminal end edges **60, 62**. The crotch member **50** bridges the gap between the terminal edges **14, 22** of the body panels **4**, **6** and is connected respectively to those body panels at attachment locations **88**. The crotch member 50 that overlaps the body panels or body chassis member can be minimally attached thereto with an attachment having a width of between about 10% and about 100% of the width of the absorbent insert. For example, the crotch member **50** can be attached to the body panels **4, 6** along the longitudinal side edges of the crotch member (FIGS. 8-11), or alternatively along a centerline (FIGS. 6 and 7) such that the edges of the absorbent insert are not attached to the body panels or body chassis. In another embodiment, the entirety of the portion of the crotch member that overlaps the body panels can be attached thereto. The crotch member can be secured to the body panels when they are in a stretched or unstretched condition.

The attachment location can extend along the entire length of the overlapping portion of the crotch member, along only a portion of the length, or at a discrete point, for example proximate midway between the ends **60, 62** of the crotch member. In another embodiment (not shown), the attachment location can be formed from a plurality of discrete attachment locations spaced longitudinally along the centerline. In other alternatives, the terminal edges of the crotch member **50** can be attached to the body panels, or the crotch member can be secured to the body panels along the terminal crotch edges **14**, **22** thereof.

In one embodiment, shown in FIGS. 6-11, the crotch member is configured as an absorbent insert **50**, which includes a substantially liquid permeable top sheet **64**, or liner, and a substantially liquid impermeable back sheet **66**. A retention portion **70** is disposed or sandwiched between the topsheet and the backsheet, which are connected. It should be understood that the term "absorbent insert" refers to any material or assembly capable of absorbing liquids or bodily exudates, and may be formed from a single material or component, for example a retention portion, or can be formed as a composite of several components. It should also be understood that the term "crotch member" refers to any member of any material, including for example and without limitation those described herein with respect to the body panels and absorbent inserts, and is no limited to absorbent inserts and/or materials. For example, the crotch member may be made of one or more layers of a non-woven material. It should further be understood that when the crotch member does not include an absorbent material, it could still be used in conjunction with various disposable absorbent pads such as adult incontinent and/or feminine pads so as to improve the performance and comfort of those pads by maintaining them in close proximity to the body of the user.

Referring to FIGS. 6-11, the top sheet **64**, back sheet **66** and other components of the absorbent insert can be joined for example with adhesive bonds, sonic bonds, thermal bonds, pinning, stitching or any other attachment techniques known in the art, as well as combinations thereof. For example, a uniform continuous layer of adhesive, a patterned layer of adhesive, a sprayed pattern of adhesive or any array of lines, swirls or spots of construction bonds may be used to join the topsheet and backsheet, or any of the other components described herein.

In one embodiment, one or more crotch elastic members **38** are sandwiched between the top sheet and backsheet along the side edges thereof. The elastic members **38** can extend the entire longitudinal extent of the absorbent insert, or along only a portion thereof.

Additional layers, including for example, a liquid acquisition and distribution layer **72**, also referred to as a surge or transfer layer, are also preferably incorporated into the absorbent insert. In one embodiment, the transfer layer does not run the entire length of the absorbent insert and is shorter than the retention portion.

In one embodiment, the retention portion **70**, transfer layer **72** and other components, such as tissue layers, are free floating (unattached) between the back sheet **64** and the top sheet **66**, which are secured along only the peripheral edges thereof. Alternatively, the retention portion **70**, transfer layer **72** and other components are minimally attached to one or both of the back sheet **66** and top sheet **64**. For example, the retention portion can be secured to the back sheet along an attachment location positioned along the longitudinal centerline of the retention portion. Alternatively, or in combination with the back sheet connection, the transfer layer or retention portion can be minimally attached to the top sheet. In this way, the retention portion **70**, transfer layer **72** and other components do not impede or substantially affect the lateral stretchability and extensibility of the absorbent insert **50** and in particular the top sheet and back sheet, at least one of which is secured to the body chassis.

In another alternative embodiment, the retention portion is secured along the centerline at a point midway between the two ends of the retention portion. In this embodiment, the retention portion also does not restrict or impede the stretchability and extensibility of the absorbent insert, and in particular the top sheet and back sheet, in the lateral or longitudinal directions **500**, **502**.

In other embodiments, the top sheet is indirectly joined to the backsheet by affixing the topsheet to intermediate layers, such as the surge layer or retention portion, which in turn is affixed to the backsheet. The absorbent insert also may include barrier cuffs, or leakage control shields, formed along the opposite longitudinally extending edges of the absorbent composite.

In one embodiment, the back sheet **66** is a stretchable, elastic, liquid impervious member. Alternatively, the back sheet may be liquid permeable, e.g., when an additional barrier layer is used with the retention portion. In one embodiment, shown in FIG. 7, the back sheet **66** is a laminate structure made of a stretchable, elastic material, such as an elastomeric film **80**, which is laminated to an extensible non-woven material layer **82**. It should be understood that the backsheet can be formed from a single layer or substrate or more than two layers or substrates. The backsheet can be stretchable in both the lateral and longitudinal direction, or be stretchable in one direction and extensible in the other.

The backsheet **66** prevents various bodily fluids and exudates from wetting or otherwise contaminating various bedding or outer garments worn by the user over the absorbent garment. The backsheet can be made of the same materials described above in connection with the body panels. In one embodiment, the backsheet can include a film, which can be made of the various materials described above.

The backsheet may include a micro-porous, "breathable" material which permits gases, such as water vapor, to escape from the absorbent garment while substantially preventing liquid exudates from passing through the backsheet. For example, the breathable backsheet may be composed of a microporous polymer film or a nonwoven fabric which has been coated or otherwise modified to impart a desired level of liquid impermeability. For example, a suitable microporous film can be a PMP-1 material, which is available from Mitsui Toatsu Chemicals, Inc., a company having offices in Tokyo, Japan; or an XKO-8044 polyolefin film available from 3M Company of Minneapolis, Minnesota. The backsheet may also be embossed or otherwise provided with a pattern or matte finish to exhibit a more aesthetically pleasing appearance.

In various embodiments, where a component, such as the backsheet is configured to be permeable to gas while having a resistance and limited permeability to aqueous liquid, the liquid resistant component can have a construction which is capable of supporting a selected hydrohead of water substantially without leakage therethrough. A suitable technique for determining the resistance of a material to liquid penetration is Federal Test Method Standard FTMS 191 Method 5514, 1978, or an equivalent thereof.

In one embodiment, the backsheet is sufficiently impermeable to liquid and semi-liquid materials to substantially prevent the undesired leakage of waste materials, defined as exudates, including for example urine and feces. For example, the backsheet member can desirably support a hydrohead of at least about 45 centimeters (cm) substantially without leakage. The backsheet member can alternatively support a hydrohead of at least about 55 cm, and optionally, can support a hydrohead of at least about 60 cm, or more, to provide improved benefits.

In one example, the backsheet can be composed of a necked fiber, a creped fiber, a micro-pleated fiber, polymer films or the like, as well as combinations thereof. The fabrics may be woven or nonwoven materials, such as spunbond fabrics. One example of a suitable extensible material is a 60% necked, polypropylene spunbond having a basis weight of about 1.2 osy (40.69 gm⁻²). In one embodiment, the backsheet material is a three-ply laminate having inner and outer facing layers of 0.46 ounces per square yard (osy) (15.60 gm⁻²) polypropylene spunbond material (Kimberly-Clark Delta white polypropylene spunbond) and a middle layer of 10 grams per square meter (gsm) Kraton® 666 elastomer strands (3 mm apart) elongated to 4.5 to 5.5 times. The three piece laminate is adhesively laminated with 2.5 gsm Bostik Findley H2096 adhesive.

In another embodiment, the backsheet is a necked liner laminate made of a two-ply laminate consisting of a soft co-extruded film laminated to a necked nonwoven material. In particular, the material is made of a layer of 0.4 ounces per square yard (osy) (13.56 gm⁻²) polypropylene spunbond material (Kimberly-Clark Delta white polypropylene spunbond) that was necked 35% (to 65% of initial width) to a final basis weight of 0.6 osy (20.35 gm⁻²) and laminated to a Pliant Film XP-8600 0.7 mil (CaCO3 metallocene Dow Affinity and Dowlex 2035 LLDPE co-extruded film).

In another embodiment, the backsheet is a necked liner made of a nonwoven 0.4 ounces per square yard (osy) (13.56 gm⁻²) polypropylene spunbond material (Kimberly-Clark Delta white polypropylene spunbond) that was necked 65% (to 35% of initial width) to a final basis weight of 0.7 osy (23.74 gm⁻²).

In various constructions, the top sheet **64** can include various woven or nonwoven materials and laminates, which can be stretchable or extensible. In one embodiment, the top sheet **64** is an extensible material, such as a necked spunbond material. For example, the topsheet can be composed of a meltblown or spunbonded web of desired fibers, and may also be a bonded-carded web. For example, the topsheet and liner can be made of a substantially hydrophobic material, and the hydrophobic material may optionally be treated with a surfactant or otherwise processed to import a desired level of wettability and hydrophilicity. In one particular embodiment of the invention, the topsheet is a nonwoven, spunbond polypropylene fabric composed of about 2.8 - 3.2 denier fibers formed into a web having a basis weight of about 22 gsm and density of about 0.06 gm/cc. The fabric can be surface treated with an operative amount of surfactant, such as about 0.28% Triton X-102 surfactant. The surfactant can be applied by any conventional means, such as spraying, printing, brush coating or the like. In another embodiment, the top sheet can also include an elastic material, such that it is stretchable.

The retention portion 70 is made of an absorbent material, which can be any material that tends to swell or expand as it absorbs exudates, including various liquids and/or fluids excreted or exuded by the user. For example, the absorbent material can be made of airformed, airlaid and/or wetlaid composites of fibers and high absorbency materials, referred to as superabsorbents. Superabsorbents typically are made of polyacrylic acids, such as FAVOR 880 available from Stockhausen, Inc. of Greensboro, North Carolina. The fibers can be fluff pulp materials, such as Alliance CR-1654, or any combination of crosslinked pulps, hardwood, softwood, and synthetic fibers. Airlaid and wetlaid structures typically include binding agents, which are used to stabilize the structure. In addition, various foams, absorbent films, and superabsorbent fabrics can be used as an absorbent material. Various acceptable absorbent materials are disclosed in U.S. Patents 5,147,343 for Absorbent Products Containing Hydrogels With Ability To Swell Against Pressure, 5,601,542 for Absorbent Composite, and 5,651,862 for Wet Formed Absorbent Composite. Furthermore, the proportion of high-absorbency particles can range from about 0 to about 100%, and the proportion of fibrous material from about 0 to about 100%. Additionally, high absorbency fibers can be used such as Oasis type 121 and type 122 superabsorbent fibers available from Technical Absorbent Ltd., Grimsby, Lincolnshire, United Kingdom.

The retention portion preferably can be made of a single or dual layer of absorbent material. In one embodiment, the retention portion has an hour-glass shape with enlarged end regions. Alternatively, the retention portion is substantially rectangular. The retention portion can include a folded or multilayered configuration. Likewise, the entire absorbent insert can have a folded configuration, with various folds formed from one or more of the backsheet, top sheet, retention portion or other components. The retention portion can have a length substantially equal to, or slightly shorter than, the length of the absorbent insert. The retention portion can include one or more barrier layers attached to the absorbent material. In one embodiment, an upper tissue substrate **74** is disposed adjacent the retention portion. Alternatively, a lower tissue substrate **75** can be disposed adjacent an opposite side of the retention portion, or the tissue can completely envelope the retention position.

Referring to FIGS 6, 7, 10 and 11, the body side of the opposite end regions of the absorbent insert 50, and in particular, the inner, body side surface of the top sheet **64**, are connected to the garment side surface 12 of the first and second body panels **4, 6** at attachment locations **88**. In an alternative embodiment, shown FIGS. 8 and 9, the absorbent insert, and in particular the garment side thereof, is connected to the body side surface **10** of the first and second body panels. It should be understood that the absorbent insert **50** can be secured using any of the methods of attachment described above, including for example various adhesives, stitching or other bonding methods. The absorbent insert can be secured to the body panels with any configuration of attachment lines, swirls, patterns, spots, etc., or can be a full and continuous attachment therebetween.

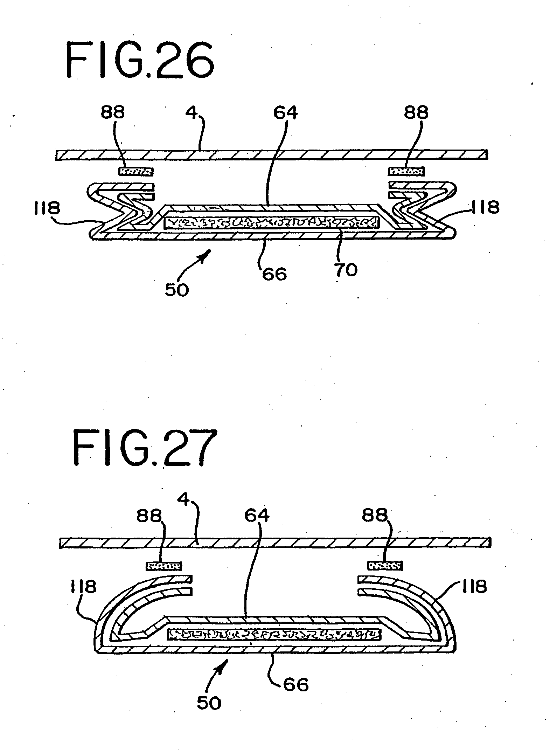

Referring to FIGS. 26 and 27, the crotch member **50**, and in particular one or more of the top sheet **64**, back sheet **66,** and retention portion **70**, are pleated and formed with at least a pair of folds **118**, which allow the crotch member 50 to unfold as the body panel material is stretched or elongated. The folds can be Z-shaped (FIG. 26), C-shaped (FIG. 27), or have any other configuration.

In one embodiment, the crotch member **50** is secured to the body panel **4, 6** material after the body panel material is elongated. In this way, the body panel material can be relaxed so as to gather the crotch member.

In another embodiment, the crotch member is minimally attached to the body panel, for example along an attachment line in line with the slit at the centerline of the crotch member. In this way, the crotch member does not inhibit or restrict the ability of the body panel to stretch.

In another embodiment, as one or both of the body panel members **4, 6** are elongated, with the attendant application of a tensile force, the absorbent insert, which includes in one embodiment an extensible top sheet **64** and a stretchable/elastic back sheet **66**, stretches or elongates with the body panels.

The back sheet 66 can be elongated in these various embodiments between about 20% and about 150%, between about 40% and about 125% and between about 50% and about 100%. Since, in one embodiment, the retention portion **70** is free floating, or alternatively is minimally attached to the stretchable top sheet and the stretchable/elastic back sheet, e.g., along the longitudinal center line, the retention portion 70 does not restrict the elongation of the back sheet **66** and top sheet **64**, or the attached body panels **4, 6**.

As shown in FIGS. 6 and 7, an outer cover 102 can be secured to the garment side surface **12** of the front body panel **4** over the end portion **60** of the crotch member **50** so as to improve the aesthetics thereof. In one embodiment, the outer cover has substantially the same shape as the front body panel, and has a length less than the overall length of the undergarment measured between the opposite waist edges thereof. In other embodiments, the outer cover has a lesser area than the front body panel, but is sufficient to cover the end portion of the absorbent insert. In this embodiment, the outer cover can have various shapes including without limitation a rectangular, circular, trapezoidal or square shape. The outer cover **102** is preferably made of a non-woven material, for example, a spun-bond material. In other embodiments, an outer cover is also secured to the garment side surface of the rear body panel over the end portion of the absorbent insert attached thereto. In yet another embodiment, an outer cover extends substantially the length of the undergarment and is secured to the garment side surfaces of both the front and rear body panel.

Referring to FIGS. 8-13, in one embodiment, the crotch edges **16, 24** of the front and rear body panels have a longitudinally extending slit **104** formed therein. In other embodiments, a plurality of spaced apart slits are formed in the crotch edge. The slit **104** is covered by the crotch member **50**, which is connected to one of the garment side or body side **10,12** of the body panels **4, 6**. The front and rear body panels each have a length Lf, Lr, while the slit in each has a length Lsf, Lsr. In various embodiments the length of the slits Lsf, Lsr are between about 5% and about 75% of the body panel lengths Lf and Lr respectively. In other embodiments, the length of the slits Lsf, Lsr are between about 10% and about 50% of the body panel lengths Lf and Lr respectively. While in other embodiments, the length of the slits Lsf, Lsr are between about 15% and about 30% of the body panel lengths Lf and Lr respectively. It should be understood that the slits can be formed in only one of the front and rear body panels. In other embodiments, a plurality of longitudinally extending slits are formed in one or both of the front and rear body panel crotch edges. In various embodiments the slit has a length Lsf, Lsr of between about 0.10 inches (0.25 cm) and 1.50 inches (3.81 cm), between about 0.25 inches (0.64 cm) and about 1.50 inches (3.81 cm), between about 0.50 inches (1.27 cm) and about 1.00 inches (2.54 cm), or about 0.75 inches (1.91 cm).

Referring to FIG. 6, the crotch edge **16, 24** of each of the front and rear body panels has a cut-out **106** formed therein. The cut-out **106** extends generally inward from a plane defined by the innermost crotch portion, and in one embodiment has a generally concave shape. The cut-out can be circular, semicircular, oval shaped, half oval-shaped, rectangular, triangular, diamond, hexagonal, pentagonal, trapezoidal or any other shape. In this way, the cut-out 106 provides access to the crotch member **50** lying therebeneath (when attached to the garment side surface of the body panel), or provides room for the crotch member **50** to expand outwardly away from the body of the user (when attached to the body side surface of the body panel). The slits **104** formed in the body panels shown in FIGS. 8-11 perform the same function. It should be understood that the cut-out **106** may be formed in only one of the front and rear body panels. In other embodiments, one of the body panels has a slit, while the other has a cutout. In yet another embodiment, the body panel has a cutout, with a slit extending from the edge of the cutout. In yet another embodiment, the material removed to form the cut-out(s) is not completely separated from the body panel(s), but rather is folded over the body panel(s) to provide reinforcement, for example at the region where the crotch member is attached.

In operation, the user applies the undergarment to their body, whether by way of pulling it up around their waist as a pant-like garment or by way of fastening it about their waist with fasteners as a diaper-like garment. As the garment is applied or fitted to the body of the user, the front and rear body panels **4, 6** are elongated from a first condition, preferably relaxed, to a second condition, preferably elongated, in at least one direction, preferably the lateral direction **502**. Of course, the body panel members can also elongate in the longitudinal direction 500 from the crotch to the waist. In one embodiment, one or both of the body panels **4, 6** is elongated in a lateral direction **502** between about 20% and about 300%, in another embodiment between about 50% and about 200%, and in another embodiment between about 100% and about 150%, as it is applied to the user. The body panel members are elongated by virtue of a tensile force being applied thereto as they conform to the body of the user.

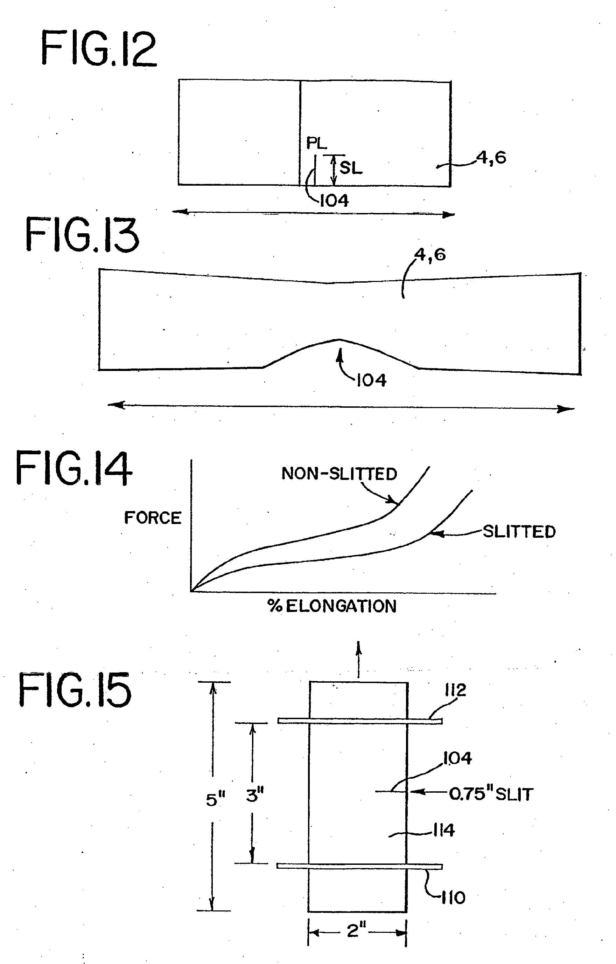

Referring to FIGS. 12-14, when a body panel **4,6** having a slit **104** formed therein is elongated in a direction substantially perpendicular to the slit, the body panel can be more easily elongated. In essence, the force required to elongate the body panel is reduced. In this way, the undergarment can be made to more easily conform to the body of the user and does not provide an overly restrictive feel to the user. It should be understood that the cut-out can serve the same function.

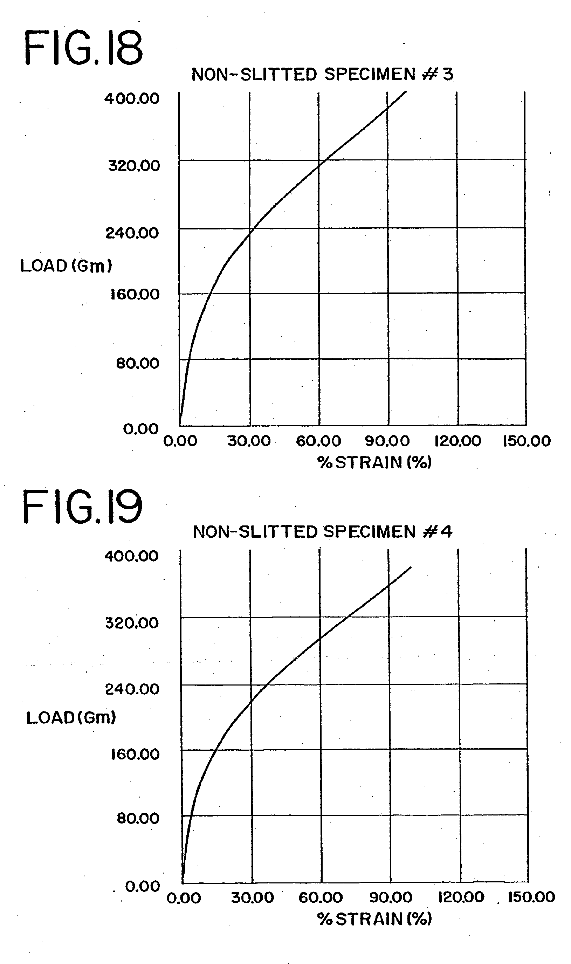

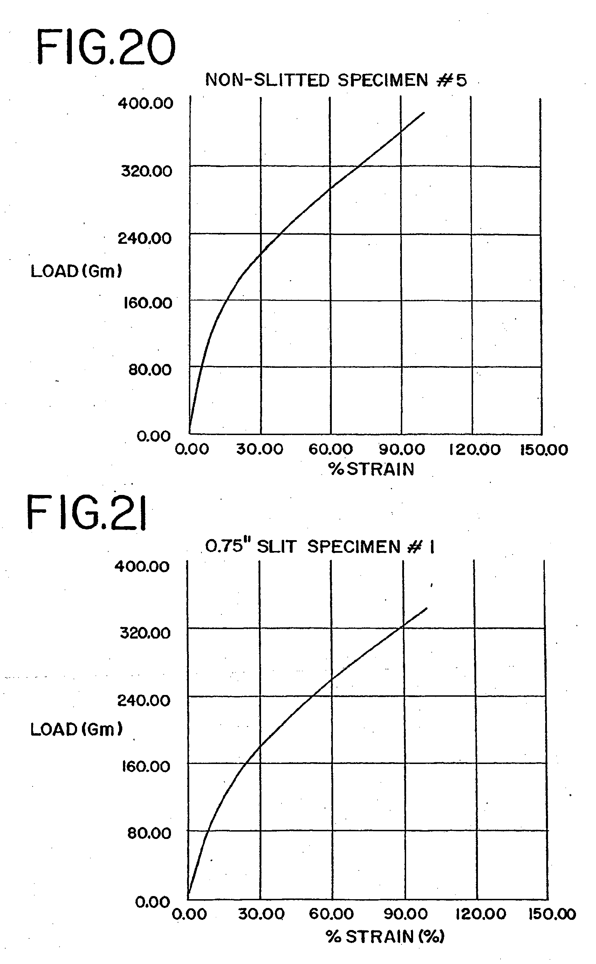

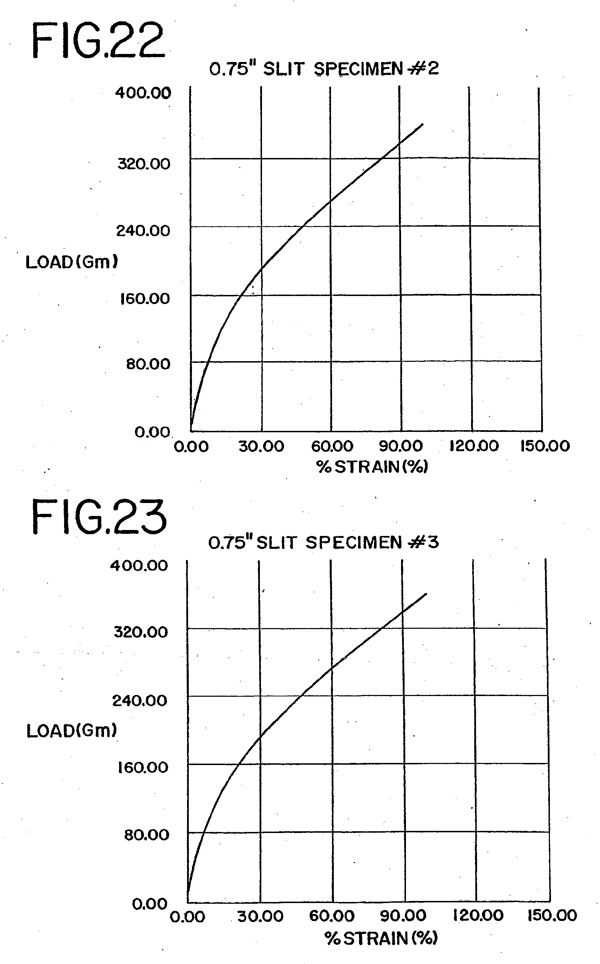

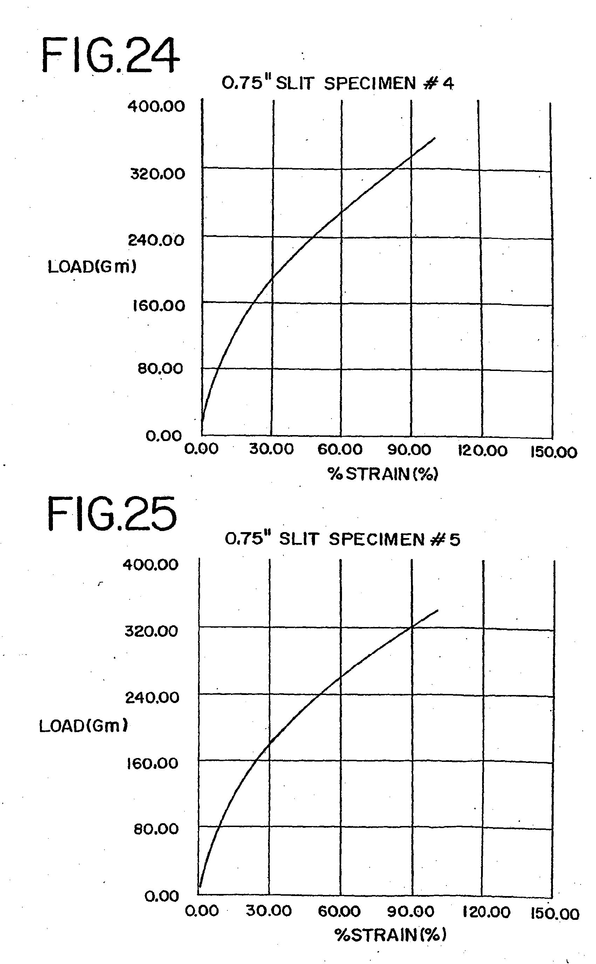

Referring to FIGS. 15-25, a body panel material was tested to determine the effect of a slit on the force required to elongate the material. In particular, a VFL body panel material was tested. The material is a three-ply laminate having inner and outer facing layers of 0.46 ounces per square yard (osy) (15.6 gm⁻²) polypropylene spunbond material (Kimberly-Clark Delta white polypropylene spunbond) and a middle layer of 10 grams per square meter (gsm) Kraton® 666 elastomer strands (3 mm apart) elongated to 4.5 to 5.5 times. The three piece laminate is adhesively laminated with 2.5 gsm Bostik Findley H2096 adhesive.

The test procedure for testing the body panel material is as follows:
I. Sample Preparation:
   1. Ten specimens **114** of 2 inches (5.08 cm) wide by 5 inches (12.70 cm) long were cut from a sheet of VFL material. The stretchable direction of the material is the length direction of the specimens, which is also the test direction.
   2. A slit **104** of 0.75 inches (1.91 cm) was cut in five of the specimens, as shown in FIG. 15.
II. Tensile Test:
   1. The tensile test was conducted on a tensile tester (Model: Synergie 200 available from MTS located at 14000 Technology Drive, Eden Prairie, MN) located in an environmental room where the temperature was kept at 23 degree C and the relative humidity was kept at 50%.
   2. The initial distance between the lower (stationary) and the upper (moving) jaws **110, 112** of the tensile tester was set at 3 inches (7.62 cm), as shown FIG. 15.
   3. The specimen was clamped onto the jaws, with the slit located half way between the two jaws.
   4. The moving (upper) jaw was activated to travel at a constant speed of 10 inches/min away the stationary (lower) jaw. The moving jaw was stopped at an extension of 3 inches (100% extension). The load limit was 10 kg.
   5. The load (grams) v. % strain curve was recorded on a computer equipped with TestWorks Version 3.10 software program available from MTS.
   6. The load (grams) at 100% strain and the total energy (gram-cm) applied to the specimen during the stretching were also recorded.
   7. Five specimens without a slit and five specimens with a slit were tested.

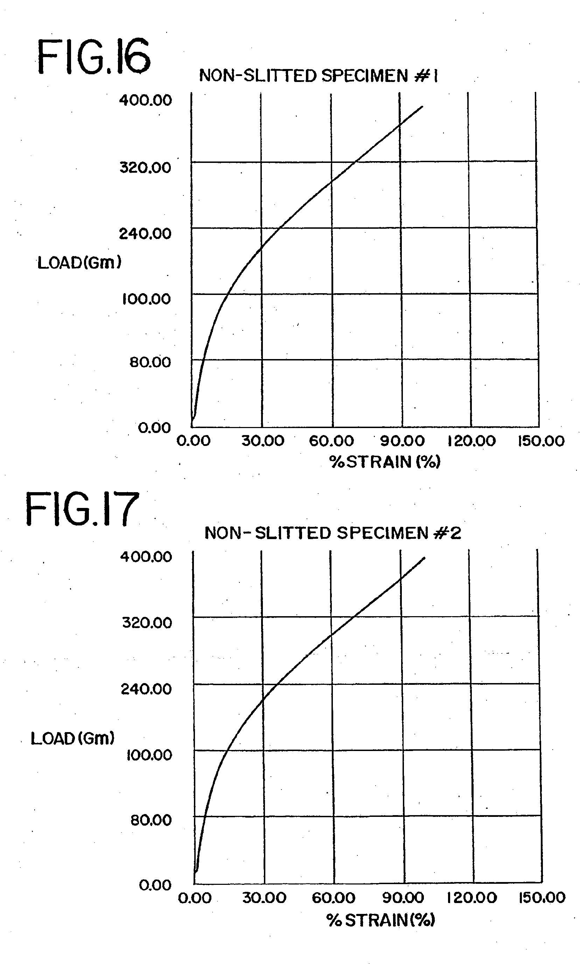

The load v. % strain curves for the ten specimens are shown in FIGS. 16-25. In addition, the Load at 100% Strain (gm) and Total Energy (gm-cm) for each sample are shown in Tables 1 and 2.

**Table 1: Unslit Specimens**

| | **Load at 100% Strain Gm** | **Total Energy Gm-cm** |
|---|---|---|
| 1 | 382.9 | 1955.89 |
| 2 | 389.3 | 1978.08 |
| 3 | 402.8 | 2078.56 |
| 4 | 378.5 | 1944.97 |
| 5 | 380.4 | 1950.50 |
| | | |
| | | |
| Mean | 386.8 | 1981.60 |
| Min | 378.5 | 1944.97 |
| Max | 402.8 | 2078.56 |
| StdV | 9.8 | 55.64 |
| %Cov | 2.5 | 2.81 |

**Table 2: Slit Specimens**

| | **Load at 100% Strain Gm** | **Total Energy Gm-cm** |
|---|---|---|
| 1 | 344.0 | 1680.55 |
| 2 | 358.8 | 1760.50 |
| 3 | 359.7 | 1782.71 |
| 4 | 356.5 | 1765.68 |
| 5 | 339.3 | 1664.52 |
| | | |
| | | |
| Mean | 351.6 | 1730.79 |
| Min | 339.3 | 1664.52 |
| Max | 359.7 | 1782.71 |
| StdV | 9.4 | 54.11 |
| %Cov | 2.7 | 3.13 |

When looking at the data shown in FIGS. 16-25 and Tables 1 and 2, it is readily apparent that the loads for the slit specimens are lower than the loads for the non-slit specimens at any given strain. This finding indicates that it is easier to stretch an elastic panel with a slit.

In addiction, the average load at 100% strain for the non-slit specimens is about 387 grams while the average load at 100% strain for the slit specimens (0.75 inch (1·91 cm) slit) is about 352 grams. This represents about a 10% difference in the force required to stretch a slit and a non-slit specimen.

Moreover, the average total energy required to stretch the non-slit specimens to 100% is about 1982 grams-cm while the average total energy requires to stretch the slit specimens to 100% is about 1731 grams-cm. This represents about 14% difference in the energy required to stretch a slit and a non-slit specimen.

In another aspect, the manufacturer or retailer of the afore-described absorbent garments provides instructional information to the user, for example by way of textual or pictorial indicia on the packaging materials, about how the garment works. For example, the manufacturer or retailer can explain to the end user the advantages of the stretchable/elastic absorbent insert, the slit and/or the cutout, and the resultant ability of the body panels to freely conform to the body of the user without restriction from the retention portion, thereby improving the conformance and fit of the garment.

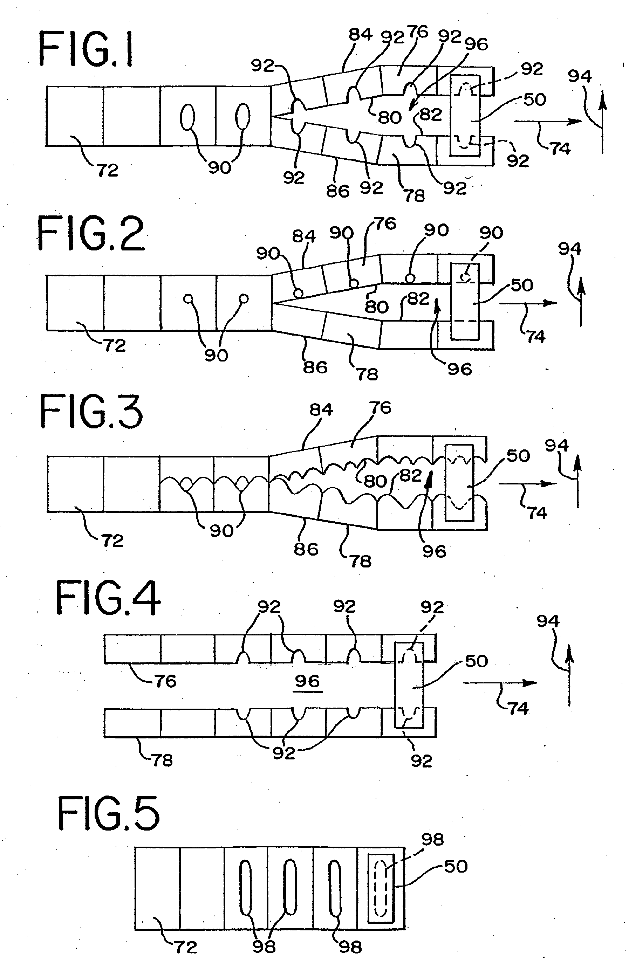

Referring to FIGS. 1-3, the method for fabricating one or more embodiments of the aforedescribed undergarments is illustrated. In particular, a web **72** of body panel material is cut in a longitudinal machine direction **74** to form a front and rear body panel web **76, 78** each having a cut edge **80, 82** and an outer lateral edge **84, 86**. In one embodiment, as shown in FIGS. 1 and 2, the cut edges **80, 82** are linear. In another embodiment, shown in FIG. 3, the cut edges **80, 82** have a non-linear profile, and can be formed for example as undulating wave pattern. In one embodiment, the web is cut in a sinusoidal wave pattern, which should be broadly interpreted as a pattern having peaks and valleys. The pattern can be formed of undulating curves or wave patterns, or can include or be made entirely of various linear portions. In this way, the front body panel can be provided with a different shape than the rear body panel.

Referring to the embodiments of FIGS. 1 and 2, a die cutter is used to successively make a plurality of cutouts **90** spaced apart in the machine direction. The cutouts **90** can have a multitude of shapes, as explained above, including the circular shaped shown in FIG. 2, the oval, or egg shape shown in FIG. 1, or the semi-circular, half oval-shaped, rectangular, triangular, diamond, hexagonal, pentagonal, trapezoidal or any other shape as explained above. After the cutout **90** is made, the web is cut along the machine direction to form the cut edges **80,82** as just explained. The cut can be made such that the cut edges **80,82** intersect the cutout **90**, as shown in FIG. 1, such that each of the front and rear body panel webs **76, 78** has a cutout **92,** or it can be offset on one side of the cutout **90** such that only one of the body panel webs has a cutout **90** as shown in FIG. 2. In such an embodiment, the front and rear body panels can be provided with different strain and elongation properties.

Referring to FIG. 3, the cut edges **80, 82** and cutout can be formed simultaneously, for example with a die cutter.

Alternatively, as shown in FIG. 4, the web is first cut to form a front and rear body panel web **76, 78**, or two webs are fed independently in the machine direction **94**, and a cutout **92** is formed in each web independently, albeit in one embodiment by a single cutter device.

In all of the embodiments of FIGS. 1-4, the front and rear body panel webs **76, 78** are also separated, or shifted, outwardly relative to one another in the lateral cross-direction 9**4** so as to form a gap **96** between the cut edges **80, 82** or the front and rear body panel webs **76, 78**. If, in FIG. 4, the webs **76, 78** are fed independently into the machine in a spaced apart relationship, then no shifting is required. Referring to FIGS. 1-4, the crotch member **50** is applied over the cutouts **90, 92** and is secured to one of the body side or garment side surfaces of the body panel webs **76, 78**. The webs **76, 78** can be thereafter cut-in the cross-machine direction, as is well know in the art, to form the individual undergarments.

Referring to FIG. 5, in a method not according to the present invention, a cutout is formed in a body panel web 72, which is not slit or otherwise cut in the machine direction and generally extends from one waist edge of the garment to the other. A crotch member **50** is applied over and covers the cutout **98** as it is secured to one of the bodyside or garment side surfaces of the body panel web **72**. When attached to the body side surface of the body panel, the crotch member, which may be configured as an absorbent insert, expands through the cutout 98 or opening as it is insulted during use.

In various embodiments, the spacing between the cut edges **80, 82** of the respective front and rear body panels is between about 10 mm and about 800 mm, between about 50 mm and about 500 mm, or between about 100 mm and about 300 mm. In an alternative embodiment, the cut edges **80, 82** and the crotch portions of the front and rear body panels overlap, and can be secured one to the other. In such an embodiment, the panels can be separated slightly, or can simply be shifted in the longitudinal direction without any lateral separation.

Cross-machine direction slits **104** formed in the front and rear body panels can be formed in the same way as the cut-outs 90, 92 described above. In particular, the slits **104** can be first formed in a web, which is thereafter cut in the longitudinal machine direction through the slits to form front and rear body panel webs each having a slit extending transversely to the cut edges. Alternatively, the web can first be cut in the machine direction, with the slits thereafter formed in the cross-machine direction, before or after the front and rear body panel webs are separated: In yet another alternative, the slits and cut edges are formed simultaneously.

The cutouts **90, 92** and slits **104** can be formed in the body panel webs, which are elastic, while they are in either a stretched or relaxed condition. Likewise, the crotch members **50** can be secured to the body panel webs **72, 76, 78** while the body panel webs are in a stretched or relaxed condition. The edges formed by the slit are substantially abutted when the body panel webs are in the relaxed condition, while they are separated when the body panel webs are in the stretched condition. The slits and cutouts can be made through the leg elastic elements, which may be disposed along the entirety of the cut edges on both the front and rear body panel webs. If the leg elastics are in tension, this may tend to open the slit or cutout after the leg elastics are severed. The leg elastics can be deadened or otherwise omitted or removed to avoid such separation.

It should be understood that the body panel webs can be cut to form the cut edges, separated in the cross-direction, and also shifted in the longitudinal direction, as disclosed for example and without limitation in U.S. Patent Application No. 10/261,805, filed October 1, 2002 and entitled "Three-Piece Disposable Undergarment and Method in the Manufacturing Thereof,". The slits or cutouts in the body panel web that is shifted forwardly relative to the other body panel web, are aligned with the next positioned slit or cutout in the other body panel web, such that each undergarment has a slit or cutout formed in each of the front and rear body panel webs. Of course, it should be understood that only one of the front and rear body panel webs may be provided with a cutout or slit.

## Claims

1. A disposable undergarment (2) comprising:
a front body panel (4) comprising a pair of opposite laterally spaced first side edges (30,32), a first waist edge (20) and a first crotch edge (16) longitudinally spaced from said first waist edge (20); and
a rear body panel (6) comprising a pair of opposite laterally spaced second side edges (30,32), a second waist edge (28) and a second crotch edge (24) longitudinally spaced from said second waist edge (28),
said first and second crotch edges (16,24) are longitudinally spaced from each other,
**characterised in that**:
at least one of said first and second crotch edges (16,24) comprises at least one longitudinally extending slit (104) formed therein; and
the disposable undergarment (2) further comprises a crotch member (50) connected to said front and rear body panels (4,6) and covering said at least one slit (104).

2. The disposable undergarment (2) of claim 1, wherein said first and second crotch edges (16,24) comprise first and second longitudinally extending slits (104) formed therein respectively, wherein said crotch member (50) covers said first and second slits (104).

3. The disposable undergarment (2) of claim 1 or 2, wherein said at least one of said front and rear body panels (4,6) comprising said at least one slit (104) comprises an elastic material and is expandable between at least a first and second condition, wherein a pair of edges defining said slit (104) are substantially abutted when said at least one of said front and rear body panels (4,6) comprising said elastic material are in said first condition, and wherein said pair of edges defining said slit are separated when said at least one of said front and rear body panels (4,6) comprising said elastic material are in said second condition.

4. The disposable undergarment (2) of claim 3, wherein said crotch member (50) is connected to said at least one of said front and rear body panels (4,6) comprising said at least one slit (104) when said at least one of said front and rear body panels (4,6) comprising said slit (104) is in one of said first and second conditions.

5. The disposable undergarment (2) of claim 1, wherein said front body panel (4) has a first length (L_{f}) defined between said first waist edge (20) and said first crotch edge (16), and wherein said rear body panel (6) has a second length Lᵣ defined between said second waist edge (28) and said second crotch edge (24), and wherein said slit (104) has a third length Lₛᵣ, wherein said third length Lₛᵣ is between about 5% and about 75% of at least one of said first and second lengths (L_{f},Lᵣ).

6. A method of manufacturing the disposable undergarment of claim 1, comprising:
moving a web (72) of body panel material in a longitudinal machine direction (74);
forming a cross-machine direction slit (90) in said web (72);
connecting said crotch member (50) to said web (72), wherein said crotch member (50) extends in said cross-machine direction (94) and covers said slit (104); and
cutting said web (72) along said longitudinal machine direction (74) and thereby forming said front body panel web (76) and said rear body panel web (78).

7. The method of claim 6, wherein said cutting said web (72) comprises cutting said web (72) through said slit (104) after forming said cross-machine direction slit (104), wherein each of said front and rear body panel webs (76,78) comprises a longitudinally extending cut edges (80,82) and a cross-direction slit (104) extending laterally from said longitudinally extending cut edge (80,82) after said cutting said web (72).

8. The method of claim 6 or 7, further comprising stretching said web (72) in said longitudinal direction (74) prior to said forming said cross-machine direction slit (104) in said web (72).

9. The method of any of claims 6 to 8, further comprising stretching said web (72) prior to said connecting said crotch member (50) thereto.

10. A disposable undergarment (2) comprising:
a front body panel (4) having a body side surface (10) and a garment side surface (12) and comprising a pair of opposite laterally spaced first side edges (30,32), a first waist edge (20) and a first crotch edge (14) longitudinally spaced from said first waist edge (20); and
a rear body panel (6) having a body side surface (10) and a garment side surface (12) and comprising a pair of opposite laterally spaced second side edges (30,32), a second waist edge (28) and a second crotch edge (22) longitudinally spaced from said second waist edge (28), wherein:
said first and second crotch edges (14,22) are longitudinally spaced from each other;
at least one of said front and rear body panels (4,6) has a cutout (106) formed therein adjacent respectively at least one of said first and second crotch edges (14,22); and
the disposable undergarment (2) further comprises a crotch member (50) connected to said garment side surfaces (12) of said front and rear body panels (4,6) and covering said cutout (106),
**characterised in that** said disposable undergarment further comprises:
an outer cover (102) having a length less than a length of said undergarment (2) measured between said respective first and second waist edges (30,32), said outer cover (102) secured to said garment side surface (12) of said at least one of said front and rear body panels (4,6) having said cutout (106) formed therein and covering at least a portion of said crotch member (50) connected thereto, wherein said outer cover (102) is secured to said garment side surface (12) of said front body panel (4) and said cutout (106) is formed in said front body panel (4), said outer cover (102) covering an end portion of said crotch member (50) connected to said garment side surface (12) of said front body panel (4).

11. The disposable undergarment (2) of claim 10, wherein said outer cover (102) has substantially the same shape as said front body panel (4).

12. The disposable undergarment (2) of claim 10, wherein said outer cover (102) has a lesser area than said front body panel (4), but is sufficient to cover the end portion of said crotch member (50).

13. The disposable undergarment (2) of any of claims 10 to 12, wherein each of said front and rear body panels has a cutout (106) formed therein.

14. A method of manufacturing the disposable undergarment of claim 10, comprising:
moving a web (72) of body panel material in a longitudinal machine direction (74);
forming said cutout (90) in said web (72);
cutting said web (72) of body panel material along said longitudinal machine direction (74) and thereby forming a rear body panel web (78) and a front body panel web (76); and
connecting said crotch member (50) to each of said rear and front body panel webs (76,78), wherein said crotch member (50) covers said cutout (106).

15. The method of claim 14, wherein said cutting said web (72) comprises cutting said web (72) such that said cutout (90) is formed entirely in one of said front and rear body panel webs (76,78).

16. The method of claim 15, wherein said cutting said web (72) comprises cutting said web (72) such that a portion of said cutout (90) is formed in each of said front and rear body panel webs (76,78).

17. The method of any of claims 14 to 16, further comprising stretching said web (72) in said longitudinal direction (74) prior to said forming said cutout (90) in said web (72).

18. The method of any of claims 14 to 17, further comprising separating said front and rear body panel webs (76,78) prior to connecting said crotch member (50) thereto.

19. The method of any of claims 14 to 18, further comprising stretching at least one of said front and rear body panels webs (76,78) prior to said connecting said crotch member (50) thereto.

## Patentansprüche

1. Einwegunterwäsche (2), welche umfasst:
eine vordere Körperbahn (4), welche ein Paar von gegenüberliegenden seitlich beabstandeten ersten Seitenrändern (30, 32), eine ersten Taillenrand (20) und einen ersten Schrittrand (16) umfasst, der längs beabstandet ist von dem ersten Taillenrand (20); und
eine hintere Körperbahn (6), welche ein Paar von gegenüberliegenden seitlich beabstandeten zweiten Seitenrändern (30, 32), eine zweiten Taillenrand (28) und einen zweiten Schrittrand (24) umfasst, der längs beabstandet ist von dem zweiten Taillenrand (28);
wobei der erste und zweite Schrittrand (16, 24) der Länge nach voneinander beabstandet sind,
**dadurch gekennzeichnet, dass**:
mindestens einer des ersten und zweiten Schrittrands (16, 24) mindestens einen sich der Länge nach erstreckenden Schlitz (104) darin geformt aufweist; und
wobei die Einwegunterwäsche (2) des Weiteren ein Schrittelement (50) umfasst, welches mit der vorderen und hinteren Körperbahn (4, 6) verbunden ist und den mindestens einen Schlitz (104) bedeckt.

2. Einwegunterwäsche (2) gemäß Anspruch 1, wobei der erste und zweite Schrittrand (16, 24) einen ersten beziehungsweise zweiten sich der Länge nach erstreckenden darin geformten Schlitz (104) umfassen, wobei das Schrittelement (50) den ersten und zweiten Schlitz (104) bedeckt.

3. Einwegunterwäsche (2) gemäß Anspruch 1 oder 2, wobei die mindestens eine der vorderen und hinteren Körperbahn (4, 6), die den mindestens einen Schlitz (104) umfasst, ein elastisches Material umfasst und zwischen mindestens einem ersten und zweiten Zustand dehnbar ist, wobei ein Paar von Rändern, welche den Schlitz (104) definieren, im Wesentlichen aneinander anstoßen, wenn sich die mindestens eine der vorderen und hinteren Körperbahn (4, 6), die das elastische Material umfasst, in dem ersten Zustand befindet, und wobei das Paar von Rändern, welche den Schlitz definieren, getrennt ist, wenn sich die mindestens eine der vorderen und hinteren Körperbahn (4, 6), die das elastische Material umfasst, in dem zweiten Zustand befindet.

4. Einwegunterwäsche (2) gemäß Anspruch 3, wobei das Schrittelement (50) mit der mindestens einen der vorderen und hinteren Körperbahn (4, 6), welche den mindestens einen Schlitz (104) umfasst, verbunden ist, wenn sich die mindestens eine der vorderen und hinteren Körperbahn (4, 6), welche den Schlitz (104) umfasst, in einem des ersten und zweiten Zustands befindet.

5. Einwegunterwäsche (2) gemäß Anspruch 1, wobei die vordere Körperbahn (4) eine erste Länge (L_{f}) aufweist, welche definiert ist zwischen dem ersten Taillenrand (20) und dem ersten Schrittrand (16), und wobei die hintere Körperbahn (6) eine zweite Länge Lᵣ aufweist, die definiert ist zwischen dem zweiten Taillenrand (28) und dem zweiten Schrittrand (24), und wobei der Schlitz (104) eine dritte Länge Lₛᵣ aufweist, wobei die dritte Länge Lₛᵣ zwischen ungefähr 5% und ungefähr 75% mindestens einer der ersten und zweiten Länge (L_{f}, Lᵣ) beträgt.

6. Verfahren zur Herstellung der Einwegunterwäsche gemäß Anspruch 1, welches umfasst:
Bewegen einer Bahn (72) aus Körperbahnmaterial in einer Längsmaschinenrichtung (74);
Bilden eines Schlitzes (90) in der Bahn (72) in Quermaschinenrichtung;
Verbinden des Schrittelements (50) mit der Bahn (72), wobei sich das Schrittelement (50) in der Quermaschinenrichtung (94) erstreckt und den Schlitz (104) bedeckt; und
Schneiden der Bahn (72) entlang der Längsmaschinenrichtung (74) und dadurch Bilden der vorderen Körperbahn (76) und der hinteren Körperbahn (78).

7. Verfahren gemäß Anspruch 6, wobei das Schneiden der Bahn (72) das Schneiden der Bahn (72) durch den Schlitz (104) nach dem Formen des Schlitzes (104) in der Quermaschinenrichtung umfasst, wobei jede der vorderen und hinteren Körperbahnbahn (76, 78) eine sich der Länge nach erstreckende Schnittkante (80, 82) umfasst, und wobei ein Schlitz (104) in Quermaschinenrichtung sich nach dem Schneiden der Bahn (72) seitlich von der sich der Länge nach erstreckenden Kante (80, 82) erstreckt.

8. Verfahren gemäß Anspruch 6 oder 7, welches des Weiteren das Dehnen der Bahn (72) in der Längsrichtung (74) umfasst vor dem Bilden des Schlitzes (104) in der Bahn (72) in der Quermaschinenrichtung.

9. Verfahren gemäß einem der Ansprüche 6 bis 8, welches des Weiteren das Dehnen der Bahn (72) vor dem Verbinden des Schrittelements (50) damit umfasst.

10. Einwegunterwäsche (2), welche umfasst:
eine vordere Körperbahn (4), welche eine körperseitige Oberfläche (10) und eine bekleidungsseitige Oberfläche (12) aufweist und welche ein Paar von gegenüberliegenden seitlich beabstandeten ersten Seitenrändern (30, 32), einen ersten Taillenrand (20) und einen ersten Schrittrand (14) umfasst, der der Länge nach beabstandet ist von dem ersten Taillenrand (20); und
eine hintere Körperbahn (6), welche eine körperseitige Oberfläche (10) und eine bekleidungsseitige Oberfläche (12) aufweist und welche ein Paar von gegenüberliegenden seitlich beabstandeten zweiten Seitenrändern (30, 32), einen zweiten Taillenrand (28) und einen zweiten Schrittrand (22) umfasst, der der Länge nach beabstandet ist von dem zweiten Taillenrand (28); wobei:
der erste und zweite Schrittrand (14, 22) der Länge nach voneinander beabstandet sind;
mindestens eine der vorderen und hinteren Körperbahn (4, 6) einen Ausschnitt (106) aufweist, der darin angrenzend an mindestens einen des ersten beziehungsweise zweiten Schrittrandes (14, 22) gebildet ist; und
wobei die Einwegunterwäsche (2) des Weiteren ein Schrittelement (50) umfasst, welches mit der bekleidungsseitigen Oberfläche (12) der vorderen und hinteren Körperbahn (4, 6) verbunden ist und den Ausschnitt (106) bedeckt,
**dadurch gekennzeichnet, dass** die Einwegunterwäsche des Weiteren umfasst:
eine äußere Deckschicht (102), welche eine Länge aufweist, die geringer ist als eine Länge der Unterwäsche (2), die zwischen dem jeweiligen ersten und zweiten Taillenrand (30, 32) gemessen ist, wobei die äußere Deckschicht (102) an der bekleidungsseitigen Oberfläche (12) der mindestens einen der vorderen und hinteren Körperbahn (4, 6) fixiert ist, die den Ausschnitt (106) darin gebildet aufweist, und mindestens einen Teil des Schrittelements (50) bedeckt, welches damit verbunden ist, wobei die äußere Deckschicht (102) an der bekleidungsseitigen Oberfläche (12) der vorderen Körperbahn (4) fixiert ist und der Ausschnitt (106) in der vorderen Bahn (4) gebildet ist, wobei die äußere Deckschicht (102) einen Endteil des Schrittelements (50) bedeckt, der mit der bekleidungsseitigen Oberfläche (12) der vorderen Körperbahn (4) verbunden ist.

11. Einwegunterwäsche (2) gemäß Anspruch 10, wobei die äußere Deckschicht (102) im Wesentlichen die gleiche Form aufweist wie die vordere Körperbahn (4).

12. Einwegunterwäsche (2) gemäß Anspruch 10, wobei die äußere Deckschicht (102) eine geringere Fläche als die vordere Körperbahn (4) aufweist, aber ausreicht, den Endteil des Schrittelements (50) zu bedecken.

13. Einwegunterwäsche (2) gemäß einem der Ansprüche 10 bis 12, wobei jede der vorderen und hinteren Körperbahn einen darin geformten Ausschnitt (106) aufweist.

14. Verfahren zur Herstellung der Einwegunterwäsche gemäß Anspruch 10, welches umfasst:
Bewegen einer Bahn (72) aus Körperbahnmaterial in einer Längsmaschinenrichtung (74);
Bilden des Ausschnitts (90) in der Bahn (72);
Schneiden der Bahn (72) aus Körperbahnmaterial entlang der Längsmaschinenrichtung (74) und dadurch Bilden einer hinteren Körperbahnbahn (78) und einer vorderen Körperbahnbahn (76); und
Verbinden des Schrittelements (50) mit jeder der vorderen und hinteren Körperbahnbahn (76, 78), wobei das Schrittelement (50) den Ausschnitt (106) bedeckt.

15. Verfahren gemäß Anspruch 14, wobei das Schneiden der Bahn (72) das Schneiden der Bahn (72) derart umfasst, dass der Ausschnitt (90) komplett in einer der vorderen und hinteren Körperbahnbahn (76, 78) gebildet ist.

16. Verfahren gemäß Anspruch 15, wobei das Schneiden der Bahn (72) das Schneiden der Bahn (72) derart umfasst, dass ein Teil des Ausschnitts (90) in jeder der vorderen und hinteren Körperbahnbahn (76, 78) gebildet ist.

17. Verfahren gemäß einem der Ansprüche 14 bis 16, welches des Weiteren das Dehnen der Bahn (72) in der Längsrichtung (74) umfasst vor dem Bilden des Ausschnitts (90) in der Bahn (72).

18. Verfahren gemäß einem der Ansprüche 14 bis 17, welches des Weiteren das Trennen der vorderen und hinteren Körperbahnbahnen (76, 78) umfasst vor dem Verbinden des Schrittelements (50) damit.

19. Verfahren gemäß einem der Ansprüche 14 bis 18, welches des Weiteren das Dehnen mindestens einer der vorderen und hinteren Körperbahnbahn (76, 78) vor dem Verbinden des Schrittelements (50) damit umfasst.

## Revendications

1. Sous-vêtement jetable (2), comprenant :
un panneau de corps avant (4) comprenant une paire de premiers bords latéraux opposés espacés latéralement (30, 32), un premier bord de taille (20) et un premier bord d'entrejambe (16) espacé longitudinalement dudit premier bord de taille (20) ; et
un panneau de corps arrière (6) comprenant une paire de deuxièmes bords latéraux opposés espacés latéralement (30, 32), un deuxième bord de taille (28) et un deuxième bord d'entrejambe (24) espacé longitudinalement dudit deuxième bord de taille (28),
lesdits premier et deuxième bords d'entrejambe (16, 24) étant espacés longitudinalement l'un de l'autre,
**caractérisé en ce que** :
au moins l'un desdits premier et deuxième bords d'entrejambe (16, 24) comprend au moins une fente s'étendant longitudinalement (104) formée à l'intérieur de celui-ci ; et
le sous-vêtement jetable (2) comprend en outre un élément d'entrejambe (50) relié aux dits panneaux de corps avant et arrière (4, 6) et recouvrant ladite au moins une fente (104).

2. Sous-vêtement jetable (2) selon la revendication 1, dans lequel lesdits premier et deuxième bords d'entrejambe (16, 24) comprennent des première et deuxième fentes s'étendant longitudinalement (104) formées respectivement à l'intérieur de ceux-ci, dans lequel ledit élément d'entrejambe (50) recouvre lesdites première et deuxième fentes (104).

3. Sous-vêtement jetable (2) selon la revendication 1 ou 2, dans lequel ledit au moins un desdits panneaux de corps avant et arrière (4, 6) comprenant ladite au moins une fente (104) comprend une matière élastique et est extensible entre au moins un premier état et un deuxième état, dans lequel une paire de bords définissant ladite fente (104) viennent sensiblement en butée l'un contre l'autre lorsque ledit au moins un desdits panneaux de corps avant et arrière (4, 6) comprenant ladite matière élastique est dans ledit premier état, et dans lequel ladite paire de bords définissant ladite fente sont séparés lorsque ledit au moins un desdits panneaux de bord avant et arrière (4, 6) comprenant ladite matière élastique est dans ledit deuxième état.

4. Sous-vêtement jetable (2) selon la revendication 3, dans lequel ledit élément d'entrejambe (50) est relié au dit au moins un desdits panneaux de corps avant et arrière (4, 6) comprenant ladite au moins une fente (104) lorsque ledit au moins un desdits panneaux de corps avant et arrière (4, 6) comprenant ladite fente (104) est dans l'un desdits premier et deuxième état.

5. Sous-vêtement jetable (2) selon la revendication 1, dans lequel ledit panneau de corps avant (4) a une première longueur (L_{f}) définie entre ledit premier bord de taille (20) et ledit premier bord d'entrejambe (16), et dans lequel ledit panneau de corps arrière (6) a une deuxième longueur (Lᵣ) définie entre ledit deuxième bord de taille (28) et ledit deuxième bord d'entrejambe (24), et dans lequel ladite fente (104) a une troisième longueur (Lₛᵣ), dans lequel ladite troisième longueur (Lₛᵣ) est entre environ 5 % et environ 75 % d'au moins une desdites première et deuxième longueurs (L_{f}, Lᵣ).

6. Procédé de fabrication du sous-vêtement jetable selon la revendication 1, comprenant :
le déplacement d'une toile (72) de matière de panneau de corps dans une direction de machine longitudinale (74) ;
la formation d'une fente de direction de machine transversale (90) dans ladite toile (72) ;
la liaison dudit élément d'entrejambe (50) à ladite toile (72), dans lequel ledit élément d'entrejambe (50) s'étend dans ladite direction de machine transversale (94) et recouvre ladite fente (104) ; et
la découpe de ladite toile (72) le long de ladite direction de machine longitudinale (74) en formant de ce fait ladite toile de panneau de corps avant (76) et ladite toile de panneau de corps arrière (78).

7. Procédé selon la revendication 6, dans lequel ladite découpe de ladite toile (72) comprend la découpe de ladite toile (72) à travers ladite fente (104) après la formation de ladite fente dans la direction de machine transversale (104), dans lequel chacune desdites toiles de panneau de corps avant et arrière (76, 78) comprend un bord de découpe s'étendant longitudinalement (80, 82) et une fente dans la direction transversale (104) s'étendant latéralement à partir dudit bord de découpe s'étendant longitudinalement (80, 82) après ladite découpe de ladite toile (72).

8. Procédé selon la revendication 6 ou 7, comprenant en outre l'étirement de ladite toile (72) dans ladite direction longitudinale (74) avant ladite formation de ladite fente dans la direction de machine transversale (104) dans ladite toile (72).

9. Procédé selon l'une quelconque des revendications 6 à 8, comprenant en outre l'étirement de ladite toile (72) avant ladite liaison dudit élément d'entrejambe (50) à celle-ci.

10. Sous-vêtement jetable (2) comprenant :
un panneau de corps avant (4) comportant une surface de côté de corps (10) et une surface de côté de vêtement (12) et comprenant une paire de premiers bords latéraux opposés espacés latéralement (30, 32), un premier bord de taille (20) et un premier bord d'entrejambe (14) espacé longitudinalement dudit premier bord de taille (20) ; et
un panneau de corps arrière (6) comportant une surface de côté de corps (10) et une surface de côté de vêtement (12) et comprenant une paire de deuxièmes bords latéraux opposés espacés latéralement (30, 32), un deuxième bord de taille (28) et un deuxième bord d'entrejambe (22) espacé longitudinalement dudit deuxième bord de taille (28), dans lequel :
lesdits premier et deuxième bords d'entrejambe (14, 22) sont espacés longitudinalement l'un de l'autre ;
au moins l'un desdits panneaux de corps avant et arrière (4, 6) comporte une découpe (106), formée à l'intérieur de celui-ci, adjacente respectivement à au moins l'un desdits premier et deuxième bords d'entrejambe (14, 22) ; et
ledit sous-vêtement jetable (2) comprend en outre un élément d'entrejambe (50) relié aux dites surfaces de côté de vêtement (12) desdits panneaux de corps avant et arrière (4, 6) et recouvrant ladite découpe (106),
**caractérisé en ce que** ledit sous-vêtement jetable comprend en outre :
un recouvrement extérieur (102) ayant une longueur inférieure à une longueur dudit sous-vêtement (2) mesurée entre lesdits premier et deuxième bords de taille respectifs (30, 32), ledit recouvrement extérieur (102) étant fixé à ladite surface de côté de vêtement (12) dudit au moins un desdits panneaux de corps avant et arrière (4, 6) comportant ladite découpe (106) formée à l'intérieur de celui-ci et recouvrant au moins une portion dudit élément d'entrejambe (50) relié à celle-ci, dans lequel ledit recouvrement extérieur (102) est fixé à ladite surface de côté de vêtement (12) dudit panneau de corps avant (4) et ladite découpe (106) est formée dans ledit panneau de corps avant (4), ledit recouvrement extérieur (102) recouvrant une portion d'extrémité dudit élément d'entrejambe (50) reliée à ladite surface de côté de vêtement (12) dudit panneau de corps avant (4).

11. Sous-vêtement jetable (2) selon la revendication 10, dans lequel ledit recouvrement extérieur (102) a sensiblement la même forme que ledit panneau de corps avant (4).

12. Sous-vêtement jetable (2) selon la revendication 10, dans lequel ledit recouvrement extérieur (102) a une aire qui est inférieure à celle dudit panneau de corps avant (4) mais qui est suffisante pour recouvrir la portion d'extrémité dudit élément d'entrejambe (50).

13. Sous-vêtement jetable (2) selon l'une quelconque des revendications 10 à 12, dans lequel chacun desdits panneaux de corps avant et arrière comporte une découpe (106) formée à l'intérieur de celui-ci.

14. Procédé de fabrication du sous-vêtement jetable selon la revendication 10, comprenant :
le déplacement d'une toile (72) de matière de panneau de corps dans une direction de machine longitudinale (74) ; la formation de ladite découpe (90) dans ladite toile (72) ;
la découpe de ladite toile (72) de matière de panneau de corps le long de ladite direction de machine longitudinale (74) en formant de ce fait une toile de panneau de corps arrière (78) et une toile de panneau de corps avant (76) ; et
la liaison dudit élément d'entrejambe (50) à chacune desdites toiles de panneau de corps arrière et avant (76, 78), dans lequel ledit élément d'entrejambe (50) recouvre ladite découpe (106).

15. Procédé selon la revendication 14, dans lequel ladite découpe de ladite toile (72) comprend la découpe de ladite toile (72) de sorte que ladite découpe (90) soit formée entièrement dans l'une desdites toiles de panneau de corps avant et arrière (76, 78).

16. Procédé selon la revendication 15, dans lequel ladite découpe de ladite toile (72) comprend la découpe de ladite toile (72) de sorte qu'une portion de ladite découpe (90) soit formée dans chacune desdites toiles de panneau de corps avant et arrière (76, 78).

17. Procédé selon l'une quelconque des revendications 14 à 16, comprenant en outre l'étirement de ladite toile (72) dans ladite direction longitudinale (74) avant ladite formation de ladite découpe (90) dans ladite toile (72).

18. Procédé selon l'une quelconque des revendications 14 à 17, comprenant en outre la séparation desdites toiles de panneau de corps avant et arrière (76, 78) avant la liaison dudit élément d'entrejambe (50) à celles-ci.

19. Procédé selon l'une quelconque des revendications 14 à 18, comprenant en outre l'étirement d'au moins l'une desdites toiles de panneau de corps avant et arrière (76, 78) avant ladite liaison dudit élément d'entrejambe (50) à celles-ci.
